(19) Europäisches Patentamt
European Patent Office
Office européen des brevets

(11) **EP 2 245 944 A1**

(12) **DEMANDE DE BREVET EUROPEEN**

(43) Date de publication:
**03.11.2010 Bulletin 2010/44**

(51) Int Cl.:
***A23K 1/00*** *(2006.01)* ***A23K 1/18*** *(2006.01)*

(21) Numéro de dépôt: **10157431.7**

(22) Date de dépôt: **23.03.2010**

(84) Etats contractants désignés:
**AT BE BG CH CY CZ DE DK EE ES FI FR GB GR
HR HU IE IS IT LI LT LU LV MC MK MT NL NO PL
PT RO SE SI SK SM TR**

(30) Priorité: **30.04.2009 EP 09305387**

(83) Déclaration conformément à la règle 32(1) CBE
(solution de l'expert)

(71) Demandeur: **DANISCO A/S
1001 Copenhagen K (DK)**

(72) Inventeur: **Berger, Claudette
91540 MENNECY (FR)**

(74) Mandataire: **Cabinet Plasseraud
52, rue de la Victoire
75440 Paris Cedex 09 (FR)**

(54) **Procédé pour améliorer la digestibilité et l'assimilabilité des céréales et/ou des fibres chez un animal herbivore monogastrique**

(57) L'invention concerne un procédé pour améliorer la digestibilité et l'assimilabilité des fibres et/ou des céréales chez un animal herbivore monogastrique comprenant l'étape d'administrer audit animal herbivore monogastrique une quantité efficace d'au moins une souche de bactérie choisie dans le groupe constitué des souches de bactéries des genres *Lactobacillus, Lactococcus, Propionibacterium, Bifidobacterium et Bacillus.* L'invention concerne également un supplément alimentaire pour animal herbivore monogastrique comprenant au moins une souche de bactérie choisie dans le groupe constitué des souches de bactéries des genres *Lactobacillus, Lactococcus, Propionibacterium, Bifidobacterium et Bacillus.*

EP 2 245 944 A1

**Description**

La présente invention concerne un procédé permettant d'améliorer la digestibilité et l'assimilabilité des céréales et/ou des fibres chez un animal herbivore monogastrique.

Des microorganismes de nature variée et en quantités importantes colonisent le tractus gastro-intestinal et interagissent les uns avec les autres dans une série de relations complexes. Chez les herbivores ruminants, ces microorganismes sont essentiels dans tous les compartiments du tractus gastro-intestinal et participent notamment au bon fonctionnement de la digestion du fourrage et en particulier à la destruction des parois des cellules végétales et la digestion des fibres. Pour autant, les microorganismes naturellement présents dans le tractus gastro-intestinal des ruminants ne possèdent pas la batterie enzymatique suffisante pour réaliser une digestion complète et optimale des fibres. Ainsi, l'amélioration de la digestibilité des fibres chez les ruminants fait l'objet d'études approfondies et suscite beaucoup d'espérances. En effet, si des microorganismes, ou probiotiques, doués d'une meilleure aptitude à décomposer les fibres ou leurs composantes étaient découverts ou mis au point par génie génétique, ces microorganismes pourraient extraire du fourrage consommé une plus forte proportion des nutriments disponibles, ce qui permettrait de réduire de manière importante les rations alimentaires des ruminants en conservant un apport énergétique similaire.

US 6951643 et US 7470531 divulguent l'utilisation de *Propionibacterium* P169 chez les animaux herbivores polygastriques (ruminants) afin d'augmenter le rendement en protéine et en matières grasses dans le lait. En outre, US5534271 et US552973 divulguent quant à eux l'action combinée d'une souche produisant de l'acide lactique et d'une souche consommant de l'acide lactique pour la production de lait et de viande chez les ruminants. Enfin US6887489 et US6455063 divulguent l'utilisation de *Propionibacterium* P63 en combinaison avec une souche productrice d'acide lactique pour diminuer l'acidose chez les bovins ou réduire les diarrhées chez le porc. Cependant, aucun de ces documents ne divulgue l'administration de microorganismes capables d'améliorer le rendement énergétique chez un animal herbivore monogastrique qui a un mécanisme digestif totalement différent de celui des ruminants. En effet, alors que la digestion des ruminants est essentiellement microbienne et prend place dans le rumen, la digestion des herbivores monogastriques est essentiellement chimique et prend place, pour l'essentiel, dans l'intestin grêle et le gros intestin après que la ration alimentaire ait transité dans l'estomac. Par ailleurs, on ne peut influer sur la population microbienne du gros intestin des herbivores monogastriques, comme on le fait pour les ruminants. En effet, contrairement aux ruminants, chez les herbivores monogastriques, les microorganismes doivent transiter à travers l'estomac et l'intestin grêle avant d'arriver sur les sites où ils peuvent agir. Ainsi, la plupart des microorganismes, au contact des pH acides de l'estomac, vont être détruits. L'administration de probiotiques par voie orale chez les herbivores monogastriques est donc liée à la résistance de ceux-ci aux pH acides. Par conséquent, pour ces différentes raisons, il n'est pas possible de prévoir le comportement d'un probiotique habituellement utilisé pour les ruminants chez les herbivores monogastriques.

En outre, peu d'études ont été effectuées sur l'administration de souches probiotiques aux animaux herbivores monogastriques.

La souche probiotique *Lactobacillus pentosus WE7* qui est une souche d'origine équine, potentiellement douée de propriétés *in vitro,* s'est avérée provoquer chez les jeunes poulains, outre une augmentation d'épisodes diarrhéiques, des signes cliniques anormaux tels que l'anorexie et la dépression (J. Scott Weese and Joyce Rousseau, JAVMA, Vol. 226, No. 12, June 15, 2005).

En outre, d'autres souches de *Lactobacillus* d'origine équine ont été administrées chez des jeunes poulains dans le but d'évaluer leur efficacité dans le traitement des diarrhées cliniquement importantes (Yuyama et al. Evaluation of a host-specific Lactobacillus probiotic in neonatal foals. J Appl Res Vet Med 2004;2:26-33). Aucune de ces publications ne divulgue l'effet de microorganismes sur l'amélioration de la digestibilité et de l'assimilabilité des céréales et/ou des fibres chez l'herbivore monogastrique.

Récemment, d'autres études ont été menées chez le cheval concernant l'impact d'une levure, *Saccharomyces cerevisiae,* sur la digestibilité des fourrages et particulièrement sur la digestibilité des fibres (Glade, M. J. (1991) Journal of Equine Veterinary Science 11(1): 10-16; Glade, M. J. (1991) Journal of Equine Veterinary Science 11(6): 323-329; Glade, M. J. (1992) Supplement to proceedings of Alltech's eighth annual symposium: 1-26; Glade, M. J. and L. M. Biesik (1986) Journal of Animal Science 62: 1635-1640; Glade, M. J. and M. D. Sist (1988) Nutrition Reports International 37: 11-17; Hall, R. R., S. G. Jackson, et al. (1990) Journal of Equine Veterinary Science 10(2): 130-134; Hausenblasz, J., J. Szuco, et al. (1993) 9th Biotechnology in the Feed Industry symposium, Lexington, KY, Alltech Technical Publications; Hill, J. and S. Gutsell (1998) BSAS annual meeting, Scarborough, UK, BSAS Publ; Kim, S. M., C. M. Kim, et al. (1991) Korean Journal of Animal Nutrition and feedstuff 15(5): 272-280; Medina, B. (2003) Dijon, France, Université de Bourgogne: 159 ; Moore, B. E. and K. E. Newman (1994) Journal of Animal Science 72(Suppl 1): 261 ; Pagan, J. D. (1990) Journal of Animal Science 68(Suppl. 1): 371).

Identifier d'autres microorganismes capables d'améliorer la digestibilité et l'assimilabilité des céréales et/ou des fibres chez un animal herbivore monogastrique constitue donc un enjeu majeur pour les chercheurs.

La présente invention concerne un procédé d'amélioration de la digestibilité et de l'assimilabilité des céréales et/ou des fibres chez un animal herbivore monogastrique. En effet, de manière surprenante et inattendue, les inventeurs ont mis en évidence que certaines bactéries possédaient la propriété de favoriser la digestion des céréales et/ou des fibres de la ration alimentaire chez les animaux herbivores monogastriques. Ces bactéries appartiennent aux genres *Lactobacillus, Lactococcus, Propionibacterium, Bifidobacterium et Bacillus.* L'invention vise donc un procédé pour améliorer la digestibilité et l'assimilabilité des céréales et/ou des fibres chez un animal herbivore monogastrique comprenant l'étape d'administrer audit animal herbivore monogastrique une quantité efficace d'au moins une souche de bactérie choisie dans le groupe constitué des souches de bactéries des genres *Lactobacillus, Lactococcus, Propionibacterium, Bifidobacterium et Bacillus .*

Au sens de l'invention, la « digestibilité » est un critère qui définit le degré auquel une matière organique pourra être digérée par un animal. Dans le règne végétal (fourrages par exemple) ce critère diminue généralement avec l'augmentation du taux de lignine d'une plante. Les parties végétatives des plantes présentent des digestibilités beaucoup plus élevées que les tiges par exemple.

Par « assimilabilité », on entend, au sens de l'invention, la capacité d'un aliment d'être catabolisé en nutriments assimilables par le sang.

Au sens de l'invention, par « fibres », on entend une substance végétale naturelle constituée principalement d'un polymère carbohydrate. Des exemples non limitatifs de fibres sont les celluloses, les hémicelluloses, les pectines, les protéoglycanes, etc. Des exemples non limitatifs de sources de fibres pour les animaux herbivores sont l'herbe, le foin, la luzerne, la paille, les graines, etc., mais aussi d'autres végétaux, comme les carottes.

Au sens de l'invention, on entend par « céréale » toute plante cultivée pour ses graines et utilisable dans l'alimentation animale. On entend également spécifiquement les graines de ces plantes. Des exemples non limitatifs de céréales selon l'invention sont le maïs, le riz, le blé, l'orge, l'avoine, le seigle, le mil, le sarrasin, le quinoa et le sésame.

Plus généralement, par « digestibilité et assimilabilité des fibres et/ou des céréales », on entend le degré auquel les fibres ou les céréales pourront être digérées par l'animal en hydrates de carbone solubles, en acides gras et en acides aminés assimilables par l'animal. Une augmentation de la digestibilité et de l'assimilabilité des fibres et/ou des céréales se traduit par une augmentation de la proportion des nutriments disponibles extraite du fourrage ou des céréales consommés.

Par « animal herbivore monogastrique », on entend tout animal dont le régime alimentaire est constitué principalement de matière végétale et qui ne possède qu'une seule poche stomacale. Des exemples d'animaux herbivores monogastriques sont les équidés et les porcins.

Par « administrer », on entend l'action d'apporter au tractus gastro-intestinal de l'animal au moins une souche de bactérie selon l'invention. Plus particulièrement, cette administration est une administration par voie orale. Cette administration peut être notamment réalisée en supplémentant la ration alimentaire destinée à l'animal avec ladite au moins une souche de bactérie, la ration alimentaire ainsi supplémentée étant ensuite ingérée par l'animal. L'administration peut également être réalisée à l'aide d'une sonde gastrique ou tout autre moyen permettant d'introduire directement ladite au moins une souche de bactérie dans le tractus gastro-intestinal de l'animal.

Par « quantité efficace », on entend une quantité de bactéries suffisante pour permettre d'améliorer la digestibilité des fibres. Cette quantité efficace peut être administrée audit animal herbivore monogastrique en une ou plusieurs fois.

Par « au moins une souche », on entend une seule souche mais également des mélanges de souches comprenant au moins deux souches de bactéries.

Par « un mélange d'au moins deux souches », on entend un mélange de deux, trois, quatre, cinq, six voire plus souches.

L'invention concerne donc dans un premier aspect un procédé pour améliorer la digestibilité et l'assimilabilité des fibres et/ou des céréales chez un animal herbivore monogastrique comprenant l'étape d'administrer audit animal herbivore monogastrique une quantité efficace d'au moins une souche de bactérie choisie dans le groupe constitué des souches de bactéries des genres *Lactobacillus, Lactococcus, Propionibacterium, Bifidobacterium et Bacillus.*

Ce procédé permet à l'animal herbivore monogastrique de tirer un meilleur profit énergétique d'un aliment à base de fibres et de céréales, et par conséquent, à partir d'un même apport calorique, d'augmenter l'énergie disponible à son métabolisme. Ceci est avantageux pour l'éleveur qui peut ainsi optimiser le coût des rations alimentaires. En effet, il peut soit diminuer les rations alimentaires de l'animal pour un même apport énergétique soit diminuer la quantité de céréales riches en amidon par du fourrage plus riche en fibres et moins onéreux, ce qui lui permet une économie financière.

Ce procédé permet également à l'animal de tirer davantage d'apport énergétique de l'amidon présent dans les céréales, ce qui lui permettra d'avoir plus d'énergie mobilisable lors d'un effort (force ou vitesse). Ceci est avantageux par exemple pour les chevaux de course ou de sport.

Ce procédé permet aussi à l'animal de tirer davantage d'apport énergétique des fibres présentes dans le fourrage, ce qui lui permettra d'avoir de l'énergie mobilisable lors d'un long effort (endurance). Ceci est avantageux par exemple pour les chevaux de randonnées et de loisir.

Enfin, ce procédé permet d'augmenter l'énergie disponible pour l'animal herbivore monogastrique tout en limitant les fluctuations de sa glycémie. Ceci a pour conséquence de prolonger et stabiliser à long terme l'apport énergétique à l'animal.

Par ailleurs, alors qu'il a été observé que l'exercice physique intense, par exemple lors d'événements sportifs (courses, compétitions, ....) et les conditions de stress (par exemple lors de transport, de changement de lieu, de changement d'environnement, de changement d'entourage, de compétition, ...) entraînent des perturbations de la digestion chez les animaux (voir notamment Goachet et al., Comparative Exercise Physiology 5(3-4) ; 143-151), le procédé selon l'invention permet de corriger ces perturbations. Les animaux auxquels est administré au moins une souche de bactérie selon l'invention possèdent une digestibilité et une assimilabilité des fibres et des céréales améliorée, et cela même en conditions d'effort physique et de stress. Les souches de bactéries selon l'invention ont pour effet de prévenir les désordres digestifs.

Typiquement, de façon à avoir un effet préventif et récupérateur, les souches de bactéries selon l'invention pourront être administrées chaque jour et sur une période commençant 3 mois, 3 semaines, 2 semaines, 1 semaine ou 1 jour avant l'exercice physique intense et/ou l'évènement de stress et finissant 2 mois, 2 semaines ou 1 semaine après l'exercice physique intense et/ou l'évènement de stress.

L'invention concerne l'utilisation de souches de bactéries selon l'invention pour diminuer chez un animal herbivore monogastrique les perturbations de la digestion induites par un exercice physique intense ou un stress.

Dans un mode de réalisation particulier, le procédé selon l'invention est **caractérisé en ce que** l'étape d'administrer audit animal herbivore monogastrique une quantité efficace d'au moins une souche de bactérie choisie dans le groupe constitué des souches de bactéries des genres *Lactobacillus, Lactococcus, Propionibacterium, Bifidobacterium et Bacillus* consiste à administrer une quantité efficace d'un mélange d'au moins deux souches de bactéries choisies dans le groupe constitué des souches de bactéries des genres *Lactobacillus, Lactococcus, Propionibacterium, Bifidobacterium et Bacillus.*

De manière particulière, le mélange de souches de bactéries selon l'invention comprend au moins une souche de bactérie du genre *Lactobacillus.* Des exemples de mélanges de souches de bactéries selon l'invention sont notamment un mélange comprenant au moins deux souches du genre *Lactobacillus,* ou un mélange comprenant au moins une souche du genre *Lactobacillus* et au moins une souche du genre *Propionibacterium.*

Les proportions peuvent varier de 1% à 99%, plus avantageusement de 25% à 75% et encore plus avantageusement autour de 50% pour chaque souche. Dans un mélange comprenant plus de deux souches, les souches sont préférentiellement présentes en proportion sensiblement égales dans le mélange.

Dans encore un autre mode de réalisation de l'invention, les souches de bactéries du genre *Lactobacillus* ne sont pas des bactéries de la souche *Lactobacillus pentosus WE7.*

Selon un mode de réalisation de l'invention, les souches du genre *Lactobacillus* sont en particulier choisies parmi les espèces *L. paracasei, L. casei, L. acidophilus, L. buchnerii, L. farciminis, L. rhamnosus, L. reuteri, L. brevis, L.fermentum, L. lactis* et *L. plantarum.*

Plus particulièrement, une souche de l'espèce *L. plantarum* est la souche *L. plantarum* Lp115, déposée selon le Traité de Budapest le 9 février 2009, à la Deutsche Sammlung von Mikroorganismen und Zellkulturen GmbH (DSMZ, Inhoffenstr. 7 B, D-38124 Braunschweig, Germany) sous le numéro DSM22266 par Danisco Deutschland GmbH (Bush-Johannsen-Str. 1,25899 Niebüll, Germany). Les souches du genre *Lactococcus* sont en particulier choisies parmi les espèces *Lactococcus cremoris* et *Lactococcus lactis.*

Les souches du genre *Propionibacterium* sont en particulier choisies parmi les souches des espèces *Propionibacterium jensenii, Propionibacterium acidipropionici, Propionibacterium freudenreichii* et *Propionibacterium freudenreichii* ssp *shermanii.* Une souche particulière de l'espèce *Propionibacterium jensenii* selon l'invention est la souche *Propionibacterium jensenii* P63, déposée selon le Traité de Budapest le 15 janvier 2009, à la Deutsche Sammlung von Mikroorganismen und Zellkulturen GmbH (DSMZ, Inhoffenstr. 7 B, D-38124 Braunschweig, Germany) sous le numéro DSM22192 par Danisco Deutschland GmbH (Bush-Johannsen-Str. 1, 25899 Niebüll, Germany).

Les souches du genre *Bifidobacterium* sont en particulier choisies parmi les souches des espèces *Bifidobacterium longum, Bifidobacterium lactis* et *Bifidobacterium animalis.*

Les souches du genre *Bacillus* sont en particulier choisies parmi les souches des espèces *Bacillus licheniformis, Bacillus subtilis* et *Bacillus cereus.*

Dans un mode de réalisation de l'invention, le mélange d'au moins deux souches de bactéries est un mélange d'au moins une souche de l'espèce *L. farciminis* et d'au moins une souche de l'espèce *L. rhamnosus.*

Les mélanges de souches du genre Lactobacillus, et plus particulièrement le mélange d'au moins une souche de l'espèce *L. farciminis* et d'au moins une souche de l'espèce *L. rhamnosus,* sont particulièrement adapté aux herbivores monogastriques devant fournir un effort important sur une courte durée. En effet, comme en attestent les résultats expérimentaux de l'invention, ces mélanges particuliers de souches permettent de reproduire l'effet d'un régime énergétique riche en amidon, bien que la ration soit riche en fibres. Par conséquent, ces mélanges permettent à l'animal de tirer davantage profit de sa ration alimentaire et ce, rapidement après absorption de sa ration. Ce type de mélange est donc particulièrement adapté à des chevaux de course ou de sport.

Dans un autre mode de réalisation de l'invention le mélange d'au moins deux souches de bactéries est un mélange d'au moins une souche de *L. plantarum* et d'au moins une souche de *Propionibacterium jensenii.* En particulier, le mélange d'au moins deux souches de bactéries est un mélange de *Propionibacterium jensenii* P63 et de *L. plantarum* Lp115. Dans un mode de réalisation particulier, les souches de *Propionibacterium jensenii* (notamment P63) et de *L. plantarum* (notamment Lp115) sont présentes dans le mélange en quantité sensiblement égale (environ 50%/50%). Un tel mélange est en particulier adapté à une administration chez un herbivore monogastrique qui possède un régime pauvre en amidon et riche en fibre. En effet, comme décrit dans la partie expérimentale, ce mélange particulier de bactéries permet à l'animal de tirer davantage profit des fibres végétales et ce, sur une durée plus longue. Ce mélange est donc particulièrement bien adapté aux animaux ayant une activité endurante, comme notamment les chevaux de randonnée et de loisir.

Selon un mode de réalisation particulier de l'invention, ledit procédé pour améliorer la digestibilité et l'assimilabilité des fibres et/ou des céréales chez un animal herbivore monogastrique comprend en outre l'action d'administrer d'autres microorganismes, lesdits microorganismes étant choisis dans le groupe comprenant notamment les bactéries lactiques, les microorganismes probiotiques, les levures et les champignons (par exemple Penicillium et Geotrichum).

Selon un mode de réalisation de l'invention, les souches de bactéries sont inactivées avant leur administration à l'animal herbivore. L'inactivation permet de diminuer significativement la capacité de reproduction des microorganismes sans affecter significativement leur activité enzymatique. Typiquement, suite au procédé d'inactivation, le nombre de microorganismes capables de se reproduire est réduit d'un facteur supérieur à X, X étant choisi parmi les valeurs suivantes : $10^4$, $10^5$, $10^6$, $10^7$, $10^8$, $10^9$, $10^{10}$ et $10^{11}$.

Typiquement, les microorganismes peuvent être inactivés par un traitement par choc thermique. Par exemple, les microorganismes peuvent être exposés à des températures comprises entre 40°C et 70°C. La durée du traitement par choc thermique dépendra de la température choisie et du microorganisme devant être inactivé. Par exemple, le procédé d'inactivation peut être réalisé pendant une période de temps comprise entre 15 minutes et 96 heures. Par exemple encore, les microorganismes peuvent être exposés à des températures comprises entre 60°C et 70°C pendant une période de temps comprise entre 20 et 40 heures.

D'autres techniques peuvent être utilisées pour inactiver les microorganismes, comme par exemple l'ionisation ou la photoinactivation (inactivation par la lumière). Les microorganismes peuvent aussi être inactivés en les conservant pendant de longues périodes à une température ou à un taux d'humidité non compatible avec leur viabilité.

L'inactivation des souches de bactéries selon l'invention a pour conséquence d'empêcher la multiplication et le développement des bactéries tout en conservant leur batterie enzymatique et donc leurs propriétés de digestibilité des fibres. En outre, l'inactivation des souches permet aux souches de ne pas entrer en compétition avec la flore intestinale fibrolytique, cellulolytique et amylolytique, tout en libérant leur contenu enzymatique dans le milieu.

Selon l'invention, la digestibilité des fibres est considérée comme « améliorée » si les fibres sont mieux digérées par l'animal en présence de ladite au moins une souche de bactérie. De manière non limitative, des méthodes pouvant être utilisées pour mesurer la digestibilité des fibres sont les méthodes de dosage des produits terminaux de la fermentation. On peut citer notamment le dosage de l'acide lactique, par exemple par une méthode enzymatique colorimétrique, et le dosage des acides gras volatils (AGV), par exemple par chromatographie gazeuse comme décrit par Jouany JP et Senaud J dans Reprod Nutr Dev. 1982;22(5):735-52. Ainsi, à l'aide des tests décrits précédemment, l'homme du métier est à même de comparer la digestibilité en présence ou en absence des souches de bactéries selon l'invention.

Typiquement, ladite au moins une souche selon l'invention est administrée audit animal herbivore monogastrique pendant et/ou en dehors des prises alimentaires.

Dans un mode de réalisation particulier de l'invention, ladite quantité efficace d'au moins une souche de bactérie est administrée audit animal herbivore monogastrique en supplémentant un aliment destiné audit animal avec ladite quantité efficace d'au moins une souche de bactérie. Par « supplémenter », au sens de l'invention, on entend l'action d'incorporer directement dans l'aliment destiné à l'animal la quantité efficace de bactéries selon l'invention. Ainsi, l'animal, en se nourrissant, ingère les bactéries selon l'invention qui pourront alors jouer leur rôle d'augmenter la digestibilité et l'assimilabilité des fibres et/ou des céréales contenues dans l'alimentation de l'animal.

Ainsi, un autre objet de l'invention concerne un supplément alimentaire pour animal herbivore monogastrique comprenant au moins une souche de bactérie choisie dans le groupe constitué des souches de bactéries des genres *Lactobacillus, Lactococcus, Propionibacterium, Bifidobacterium* et *Bacillus.*

Dans un mode de réalisation de l'invention, les souches de bactéries du genre *Lactobacillus* ne sont pas choisies parmi les souches de l'espèce *Lactobacillus pentosus.*

Dans encore un autre mode de réalisation de l'invention, les souches de bactéries du genre *Lactobacillus* ne sont pas des bactéries de la souche *Lactobacillus pentosus WE7.*

Typiquement, les souches du genre *Lactobacillus* sont en particulier les souches des espèces *L. paracasei , L. casei, L. acidophilus, L. buchnerii , L. farciminis, L. rhamnosus, L. reuteri, L.fermentum, L. brevis, L. lactis* et *L. plantarum.* Plus particulièrement, une souche de l'espèce *L. plantarum* est la souche *L. plantarum* Lp115, déposée selon le Traité de Budapest le 9 février 2009, à la Deutsche Sammlung von Mikroorganismen und Zellkulturen GmbH (DSMZ, Inhoffenstr. 7 B, D-38124 Braunschweig, Germany) sous le numéro DSM22266 par Danisco Deutschland GmbH (Bush-Johannsen-Str. 1, 25899 Niebüll, Germany).

Les souches du genre *Lactococcus* sont en particulier choisies parmi les souches des espèces *Lactococcus cremoris* et *Lactococcus lactis.*

Les souches du genre *Propionibacterium* sont en particulier choisies parmi les souches des espèces *Propionibacterium jensenii, Propionibacterium acidipropionici, Propionibacterium freudenreichii* et *Propionibacterium freudenreichii* ssp *shermanii.* Une souche particulière de l'espèce *Propionibacterium jensenii* est la souche *Propionibacterium jensenii* P63, déposée selon le Traité de Budapest le 15 janvier 2009, à la Deutsche Sammlung von Mikroorganismen und Zellkulturen GmbH (DSMZ, Inhoffenstr. 7 B, D-38124 Braunschweig, Germany) sous le numéro DSM22192 par Danisco Deutschland GmbH (Bush-Johannsen-Str. 1, 25899 Niebüll, Germany).

Les souches du genre *Bifidobacterium* sont en particulier choisies parmi les souches des espèces *Bifidobacterium longum, Bifidobacterium lactis* et *Bifidobacterium animalis.*

Les souches du genre *Bacillus* sont en particulier choisies parmi les souches des espèces *Bacillus licheniformis, Bacillus subtilis* et *Bacillus cereus.*

Selon l'invention, par « au moins une souche » on entend une ou plusieurs souches de bactéries. Par « plusieurs souches » on entend un mélange d'au moins deux souches de bactéries. Dans un mode de réalisation de l'invention, ledit mélange est un mélange d'au moins une souche de l'espèce *L. farciminis* et d'au moins une souche de l'espèce *L. rhamnosus.* Dans un autre mode de réalisation de l'invention, ledit mélange est un mélange de *Propionibacterium jensenii* et de *L. plantarum.* Plus particulièrement, ledit mélange est un mélange de *Propionibacterium jensenii* P63 et de *L. plantarum* Lp115.

L'invention concerne également l'utilisation d'un supplément alimentaire pour animal herbivore monogastrique comprenant au moins une souche de bactérie choisie parmi *L. plantarum* Lp115 et *Propionibacterium jensenii* P63 telle que décrit précédemment, pour réguler la glycémie chez un animal herbivore monogastrique. En effet, ces souches particulières de bactéries ont la propriété de limiter les variations de la glycémie après la prise de la ration alimentaire : la glycémie est maintenue à un niveau plus bas, et ses fluctuations sont limitées. Cette régulation de la glycémie témoigne de la diffusion de l'apport énergétique à l'animal de manière régulière et prolongée dans le temps (cf. partie expérimentale, §2.1).

Comme décrit précédemment, et selon un mode de réalisation particulier, lesdites souches de bactéries desdits suppléments selon l'invention sont inactivées.

Selon un mode de réalisation de l'invention, ledit supplément alimentaire pour animal herbivore monogastrique comprenant au moins une souche de bactérie comprend en outre d'autres microorganismes, lesdits microorganismes étant choisis dans le groupe comprenant notamment les bactéries lactiques, les microorganismes probiotiques, les levures et les champignons (par exemple Penicillium et Geotrichum).

Un autre objet de l'invention concerne un aliment pour animal herbivore monogastrique, **caractérisé en ce qu'**il est supplémenté avec un supplément alimentaire tel que décrit précédemment.

Selon l'invention, l'aliment pour animal herbivore monogastrique pouvant être supplémenté avec ledit supplément alimentaire est typiquement choisi parmi les aliments contenant des fibres et/ou des céréales. Des exemples d'aliments selon l'invention sont l'herbe, le foin, la luzerne, la paille, les graines, ou tout autre type de fourrage utilisé pour l'alimentation des herbivores, mais également tout type d'aliment granulé, à base notamment de son de blé, d'enveloppes d'avoine, d'avoine, de luzerne, d'orge, de maïs, de marc de fruits, de mélasse de canne, de farine basse de riz, de paille, de tourteau de soja, etc. ; ou encore d'autres types de végétaux, comme notamment les carottes.

Typiquement, l'aliment est supplémenté avec ledit supplément alimentaire de manière à ce que l'animal reçoive une quantité efficace de bactéries pour améliorer la digestibilité et l'assimilabilité des fibres et/ou des céréales contenues dans l'alimentation de l'animal.

Selon la présente invention, ladite quantité efficace de ladite au moins une souche de bactérie est typiquement comprise entre $10^5$ CFU et $10^{13}$ CFU par animal et par jour, particulièrement entre $10^7$ CFU et $10^{12}$ CFU par animal et par jour, plus particulièrement entre $10^8$ CFU et $10^{11}$ CFU par animal et par jour, encore plus particulièrement d'environ $10^{10}$ CFU par animal et par jour. Lorsque les bactéries sont inactivées, les quantités décrites précédemment sont calculées avant inactivation.

De manière particulière, au sens de l'invention, ledit animal herbivore monogastrique est choisi parmi les équidés et les suidés. Plus particulièrement l'herbivore monogastrique est un équidé, typiquement choisi dans le groupe constitué des chevaux, des poneys et des ânes. De manière particulière, l'animal objet du procédé selon l'invention est le cheval ou le poney.

D'autres aspects et avantages de la présente invention sont décrits dans la figure et les exemples suivants, qui doivent être considérés à titre illustratif et comme ne limitant pas la portée de l'invention.

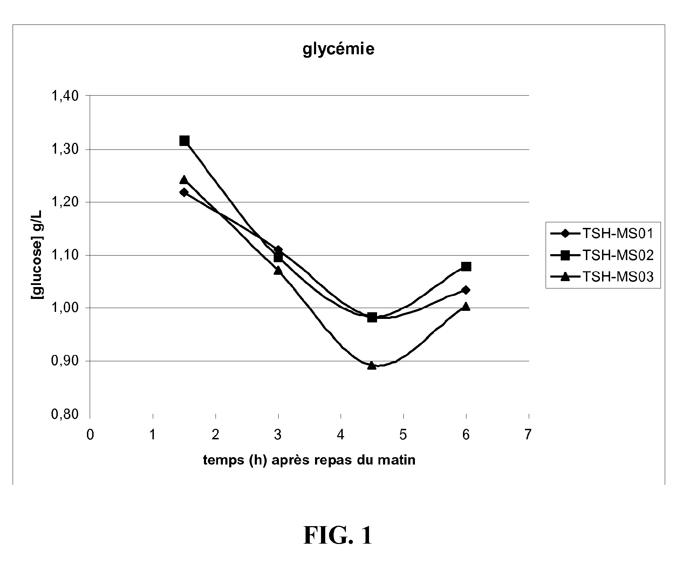

**Figure 1** : la figure représente l'évolution postprandiale de la concentration sanguine en glucose mesurée chez les chevaux recevant les traitements TSH-MS01, MS02 et MS03 (n=6).

**Exemples**

*Glossaire :*

| ADF | Acid Detergent Fiber (résidu au détergent acide) |
| ADL | Acid Detergent Lignin (résidu à l'acide sulfurique) |
| dX | digestibilité du constituant X |
| EB | Energie brute |
| g | gramme |
| kg | kilogramme |
| MB | Matière brute |
| MO | Matière organique |
| MS | Matière sèche |
| MSI | Matière sèche ingérée |
| NDF | Neutral Detergent Fiber (résidu au détergent neutre) |
| NS | Non significatif |
| PV | Poids vif |
| $R^2$ | Coefficient de corrélation |
| SD | Standard deviation (Ecart-type) |

**Exemple 1**

**1. Matériel et méthodes**

Les expérimentations décrites par la suite se sont déroulées sur la plate-forme expérimentale de l'ENESAD.

**1.1. Conduite et alimentation des animaux expérimentaux**

**1.1.1. Caractéristiques et conduite des chevaux**

Six chevaux hongres de race Trotteur Français (âge compris entre 3 et 9 ans) de poids vif initial compris entre 423 kg et 512 kg et de note d'état corporel initiale comprise entre 1 et 2 établies selon les critères des Haras Nationaux, ont été utilisés dans cet essai. Ils ont été conduits en box individuel (12,5m²), sur litière de copeaux de bois (Tier Wohl ®, Rettenmaier). Ce type de litière a permis d'éviter l'ingestion incontrôlée de fibres végétales. Ils avaient à disposition un bloc de sels minéraux à volonté (Dolmin Sel, Lactona) et avaient libre accès à un abreuvoir automatique. Les chevaux étaient à jour de leur vermifugation (Equimax, Virbac) et de leur vaccination (Equillis Prequenza TE, Intervet et Proteqflu TE, Merial).

**1.1.2. Régimes expérimentaux**

*a. Régime de base et modalités de distribution*

Les animaux ont reçu un aliment granulé en complément d'un foin de prairie (tableaux 1 et 2). La proportion 60/40 (% de MSI) a été respectée entre foin et aliment granulé. La quantité de foin et d'aliment granulé distribuée par jour a été de 1,5 kg de MB (Matière Brute) et de 1 kg de MB respectivement pour 100 kg de PV. La ration a été fractionnée en quatre apports: à 8h30 et 17h pour les granulés; à 10h et 16h pour le foin. La ration journalière des chevaux a permis de couvrir 120 % des besoins énergétiques d'entretien et de travail très léger des animaux sur la base des recommandations INRA (1990).

**Tableau 1** : Composition de l'aliment granulé DP Puissance (Evialis, France)

| Matières première |
| --- |
| Son de blé (40%) |
| Enveloppes d'avoine (10%) |
| Avoine (10%) |

(suite)

| Matières première |
| --- |
| Luzerne (10%) |
| Orge (7%) |
| Marc de fruits (7%) |
| Mélasse de canne (6%) |
| Farine basse de riz |
| Paille |
| Tourteau de soja |
| Carbonate de calcium |
| Sel |
| Bicarbonate de sodium |

**Tableau 2 :** Composition des aliments de la ration de base

| | Foin | Aliment granulé |
| --- | --- | --- |
| Matière sèche (MS), %MB | 88,95 | 89,1 |
| Matière organique (MO), %MS | 91,9 | 91,7 |
| Neutral Detergent Fiber (NDF), %MS | 64,6 | 42,0 |
| Acid Detergent Fiber (ADF), %MS | 35,9 | 20,7 |
| Acid Detergent Lignin (ADL), %MS | 4,6 | 6,0 |
| Unité fourragère cheval (UFC/kg MS) | 0,43 | 0,90 |
| Matière Azotée Cheval (MADC/kg MS) | 33,7 | 104,3 |

*b. Supplémentation en produits TSH-MS01, 02, 03:*

Les trois produits testés sont les suivants :

TSH-MS01: Mélange 50/50 de *Lactobacillus plantarum* Lp115 et *Propionibacterium jensenii* P63.
TSH-MS02: Placébo
TSH-MS03: Combinaison de deux souches inactivées de *Lactobacillus* (mélange de *L.farciminis* et *L. rhamnosus*).

Les trois produits à tester ont été préalablement mélangés à une farine de tourteau de soja et de maïs avant d'être distribués aux animaux avec le repas de granulés du matin. Les échantillons de farine portent la dénomination TSH-F1, TSH-F2 et TSH-F3. Les associations ont été faites selon le mode suivant:

- TSH-MS01 mélangé à TSH-F1
- TSH-MS02 mélangé à TSH-F2
- TSH-MS03 mélangé à TSH-F3

Les doses de produit ont été préparées dans des sachets individuels de deux grammes pour les TSH-MS, stockés à 4°C et des sachets de 50 grammes pour les TSH-F, stockés à température ambiante (< ou = 20°C). Les produits TSH-MS01 et TSH-MS03 ont été supplémenté à TSH-F1 et TSH-F3 à hauteur de $10^{10}$ CFU par cheval et par jour.

### 1.1.3. Schéma expérimental

L'essai a été réalisé selon une procédure en carré latin 3x3 (3 périodes x 3 traitements). Dans ce scénario, les deux produits TSH-MS01 et TSH-MS03 ont été testés contre un placébo sur 3 périodes expérimentales avec deux animaux par traitement (tableau 3).

Après une phase d'adaptation au régime d'une vingtaine de jours, trois périodes se sont succédé. Chaque période (environ 42 jours) était composée d'une phase de supplémentation de vingt et un jours au cours de laquelle les produits TSH-MS01 TSH-MS02 et TSH-MS03 ont été distribués et les paramètres physiologiques mesurés, puis d'une phase de washout (pas de supplémentation) pour une durée de 20 jours minimum.

Trois lots de chevaux ont été constitués de manière à ce qu'ils soient assez homogènes en fonction du poids des chevaux. Les trois lots d'animaux ont reçu alternativement un des trois traitements.

**Tableau 3** : Description du schéma expérimental

| | Adaptation (20 jours) | 1ère période (42 jours) | 2ème période (42 jours) | 3ème période (42 jours) |
|---|---|---|---|---|
| Groupe 1 C1 et C2 | Régime de base | Régime supplémenté | Régime supplémenté | Régime supplémenté |
| Groupe 2 C3 et C4 | Régime de base | Régime supplémenté TSH-MS02 | Régime supplémenté TSH-MS03 | Régime supplémenté TSH-MS01 |
| Groupe 3 C5 et C6 | Régime de base | Régime supplémenté TSH-MS03 | Régime supplémenté TSH-MS01 | Régime supplémenté TSH-MS02 |

**1.2. Etude du profil métabolique sanguin**

**1.2.1. Prélèvements et préparation des échantillons**

Pour chaque phase de supplémentation, quatre prélèvements sanguins ont été effectués pendant les six premières heures après le repas du matin pendant chaque phase de supplémentation : 1 heure et demi, 3 heures, 4 heures et demi et 6 heures en vue de déterminer les concentrations en glucose sanguin. Les prélèvements ont été effectués dans la veine jugulaire dans des tubes pré-traités au fluorure de calcium $K_2$-oxalate.

Après chaque prélèvement, le plasma a été obtenu par centrifugation (4500 trs/min, 5min) puis transféré dans un nouveau tube sec avant d'être congelés à -20°C.

**1.2.2. Analyses**

Les concentrations sanguines ont été déterminées à l'aide de kits enzymatiques du type Kit Biomérieux (kit n°61269).

**1.3. Etude de la microflore fécale et de son activité**

**1.3.1. Prélèvement et préparation des échantillons**

Pour chaque période expérimentale, les échantillons de fèces ont été prélevés 3 heures après le repas du matin par fouille rectale sur les 6 chevaux.

Une partie (10g) a été maintenue à 38°C en conditions d'anaérobiose jusqu'au moment de l'ensemencement. L'autre partie a été filtrée sur tissu nylon (porosité 100 $\mu$m). Le pH a été immédiatement mesuré sur le filtrat obtenu. Des aliquotes de 1 mL d'échantillon filtré ont été prélevés puis congelés dans des microtubes pour l'analyse ultérieure des concentrations d'acides gras volatils (AGV) et du lactate. Pour le dosage des AGV, les échantillons ont été préalablement stabilisés avec une solution contenant 100 $\mu$L d'$HgCl_2$.

**1.3.2. Mesures du pH**

Le pH a été mesuré à l'aide d'un pH-mètre de terrain (WTW, 340i, Germany) et d'électrodes adaptées.

**1.3.3. Dosage des produits terminaux de la fermentation (AGV, lactate)**

Le dosage de l'acide lactique a été réalisé selon une méthode enzymatique colorimétrique (kit Enzy plus, Diffchamb™, SE 42131 Västra Frölunda, Sweden). Le dosage colorimétrique a été effectué par spectrophotométrie d'absorption à $\lambda$=540 nm à l'aide d'un lecteur de micro-plaques (MRX revelation, Dynatech Laboratories, Guyancourt, France).

Les AGV ont été dosés par chromatographie en phase gazeuse (Gas chromatograph model 437 A, United Technologies Packard, Zurich, Suisse) selon la méthode décrite par Jouany (1982).

**1.3.4. Dénombrements microbiens**

Le dénombrement de différents groupes bactériens présents dans l'écosystème microbien fécal a été effectué selon les techniques culturales classiques (Hungate, 1969) modifiées (Grubb et Dehority, 1976) et adaptées aux chevaux (Baruc et al, 1983 ; Julliand et al, 1999). Les dénombrements exprimés en UFC ont été transformés en $\log_{10}$ UFC.

a. Préparation des *inocula*

Les *inocula* ont été préparés par dilutions décimales successives des échantillons fécaux dans un milieu de dilution en anaérobiose stricte (Bryant et Burkey, 1953). Pour chaque échantillon, trois dilutions successives étaient ensemencées, en triplicat, dans les différents milieux de culture.

b. Milieux de cultures et dénombrements

Le dénombrement de la flore anaérobie totale a été réalisé selon la technique des «roll tubes» (Hungate, 1969) sur milieu complet gélosé (Leeddle et Hespell, 1980) en anaérobiose stricte. La concentration en bactéries anaérobies totales a été déterminée après incubation à 38°C pendant 48 heures à partir des trois répétitions effectuées aux dilutions $10^{-6}$, $10^{-7}$, $10^{-8}$.

Le dénombrement de la flore cellulolytique a été réalisé en anaérobiose stricte dans des tubes contenant une bandelette de papier Whatman n° 1 comme unique substrat cellulolytique dans 4 mL de milieu (Halliwell et Bryant, 1963). Les tubes ont été ensemencés avec 1 mL d'inoculum à $10^{-4}$, $10^{-5}$, $10^{-6}$. Après 15 jours d'incubation à 38°C, le nombre le plus probable (NPP) de bactéries cellulolytiques a été estimé selon la méthode de Mac Grady.

Le dénombrement de la flore amylolytique a été réalisé en boite de Pétri par ensemencement en profondeur d'une gélose adaptée (thèse M. Varloud, 2006). Le dénombrement était réalisé après une incubation de 48 heures à 38°C à partir des trois répétitions des dilutions ($10^{-4}$, $10^{-5}$, $10^{-6}$).

Le dénombrement de la flore utilisatrice de lactate a été réalisé en anaérobiose stricte selon la méthode des «roll-tubes» sur milieu sélectif (Mackie et Wilkins, 1979). La concentration de bactéries a été déterminée après incubation à 38°C pendant 48 heures pour chaque dilution ($10^{-4}$, $10^{-5}$, $10^{-6}$).

**1.4. Mesure de la digestibilité totale apparente des constituants de la ration**

**1.4.1. Méthodologie:**

La digestibilité totale apparente (dX) du constituant X a été mesurée par récolte totale de fèces pendant 5 jours dans des harnais de digestibilité.

Elle a été calculée en utilisant l'ADL (Acid Detergent Lignin) comme marqueur interne non digéré, selon l'équation (Miraglia et al, 1999) :

$$dX = \frac{\left[(I \times Xi)/(I \times \%ADLi) - (F \times Xf)/(F \times \%ADLf)\right]}{\left[(I \times Xi)/(I \times \%ADLi)\right]}$$

avec :

Xi : la teneur en X de la matière sèche ingérée I ;
Xf : la teneur X de la matière sèche excrétée F dans les fèces ;
%ADLi : teneur en ADL de la matière sèche ingérée I ;
%ADLf : teneur en ADL de la matière sèche excrétée F ;

et dans le cas d'un régime composé fourrage/granulé :

$$I \times Xi = I_{fourrage} \times X_{fourrage} + I_{granulé} \times X_{granulé}$$

et

$$I \times \%ADLi = I_{fourrage} \times \%ADL_{fourrage} + I_{granulé} \times \%ADL_{granulé}$$

La quantité totale de fèces excrétée a été pesée chaque jour. Les éventuels refus alimentaires ont également été pesés afin de déterminer la quantité quotidienne exacte de MS ingérée.

Des échantillons de fèces, des refus alimentaires et des aliments distribués ont été prélevés chaque jour (tableau 4) et placés dans l'étuve à 65°C à circulation d'air jusqu'à l'obtention d'un poids constant afin de déterminer la MS. Une fois secs, les échantillons de fèces et de refus collectés pendant les 5 jours de digestibilité ont été broyés (grille de 0,8 mm) et rassemblés (poolés) par animal. Les échantillons d'aliments granulés et de foin ont également été broyés et cumulés en fin de période.

Tableau 4 : Grille de prélèvement des aliments distribués et refusés et des crottins lors des essais de digestibilité *in vivo*.

| Type d'échantillon | Quantité totale récoltée/jour | Quantité prélevée pour déterminer la MS |
|---|---|---|
| Aliment granulé | | 500g |
| Foin | | 1kg |
| Fèces | Moins de 10kg | 10% |
| | Plus de 10kg | 5% |
| Refus (foin et granulés) | Moins de 600g | 100% |
| | De 600g à 1500g | 50% |
| | De 1500g à 3000g | 25% |
| | Plus de 3000g | 5% |

### 1.4.2. Analyses:

Sur chaque échantillon reconstitué ont été réalisées les analyses suivantes :

- détermination de la matière sèche, à l'étuve à 65°C, jusqu'à poids constant;
- dosage des fractions pariétales (NDF, ADF, ADL) par la méthode de Van Soest (Van Soest et Wine, 1967) sur des postes semi-automatiques d'analyse fourragère (Fibertech system M 1020 Extractor, Tecator, Hoganas, Suède). Les hémicelluloses, la cellulose et l'ensemble des parois potentiellement digestibles ont alors été estimés à partir des différences NDF-ADF, ADF-ADL et NDF-ADL respectivement.
- détermination de la matière organique après passage au four (550°C pendant 5 heures).

### 1.5. Analyses statistiques

Le logiciel utilisé pour le traitement statistique de l'ensemble des données a été le logiciel SAS version 6.12 (SAS/STAT, 1998).

### 1.5.1. Données de digestibilité, de dénombrements microbiens et

### d'activité fermentaire (pH, AGV, acide lactique) :

Les données ont été traitées par analyse de variance en utilisant la procédure GLM (Generalized Linear Model). Les moyennes ajustées (Least Square means) ont été utilisées pour comparer les différences entre les traitements (fonction pdiff).

### 1.5.2. Données des concentrations sanguines (glycémie):

Les données de concentrations sanguines ont été traitées par analyse de variance (procédure GLM) à mesures répétées (repeated option du logiciel SAS) en utilisant le modèle linéaire général qui suit. Les moyennes ajustées (Least Square means) ont été utilisées pour comparer les différences entre les traitements (fonction pdiff).

### 1.5.3. Niveaux statistiques

Les niveaux statistiques retenus pour les analyses de variance ont été les suivants: $p < 0,1$ : les résultats présentent une tendance ; $p < 0,05$ : les résultats sont significativement différents ; $p < 0,01$ : la différence est très significative; $p < 0,001$ : la différence est hautement significative.

### 2. Résultats et discussion

### 2.1. Métabolisme : Glycémie

**Tableau 5:** Effet des produits TSH - MS01, 02 et 03 sur l'évolution de la concentration en glucose mesurée dans le sang des chevaux (n=6)

|  |  |  | Traitement | | | Effets (1) |
|---|---|---|---|---|---|---|
|  | SD | $R^2$ | TSH-MS01 | TSH-MS02 | TSH-MS03 |  |
| *Glycémie g/L* |  |  |  |  |  |  |
| $-T_{+1,5}$ | 0.11 | 0.88 | 1.218 | 1.317 | 1.242 | H*** |
| $-T_{+3}$ | 0.10 | 0.86 | 1.110 | 1.097 | 1.070 | (P<0.001) |
| $-T_{+4,5}$ | 0.16 | 0.83 | 0.983 | 0.983 | 0.892 | C** |
| $-T_{+6}$ | 0.06 | 0.88 | 1.033 | 1.078 | 1.003 | (p=0.003) |

(1) niveau de significativité : *p<0,05 ; **p<0,01 ; ***p<0,001
T : effet traitement ; H : effet de l'heure de prélèvement, HxT : interaction de l'heure de prélèvement et du traitement, C : effet cheval

Il est important de noter que l'analyse statistique a révélé une interaction des facteurs « heure de prélèvement » et « cheval » significative (p=0.024). Nous pouvons en déduire que l'effet individu a masqué l'effet traitement.
Bien que l'effet traitement ait été masqué par l'interaction des facteurs « effet de l'heure de prélèvement » et « effet cheval », nous pouvons clairement distinguer une tendance du produit THS-MS01 à lisser l'évolution de la glycémie, notamment à abaisser le pic de glycémie initial à $T_{+1,5}$ et à le maintenir plus bas à long terme (notamment à $T_{+6}$). Ce maintien de glycémie à un niveau plus bas, tout en limitant les fluctuations de glycémie, témoigne de la diffusion de l'apport énergétique à l'animal de manière régulière et prolongée dans le temps.

### 2.2. Microflore fécale et activité

### 2.2.1. Produits et paramètres de fermentation

**Tableau 6 :** Effet des produits TSH - MS01, 02 et 03 sur la concentration fécale en acides gras volatils totaux, les proportions molaires en acétate (C2), propionate (C3), butyrate (C4) et valérate (C5), le ratio (C2+C4)/C3, sur les concentrations en D-lactate et L-lactate et sur le ratio L/D et sur le pH mesurés dans les fèces des chevaux (n=6).

|  |  |  | *Traitement* | | | Effets (1) |
|---|---|---|---|---|---|---|
|  | $R^2$ | SD | TSH-MS01 | TSH-MS02 | TSH-MS03 |  |
| *Total AGV (mmol/L)* | 0,87 | 16,6 | 69,7 | 50,3 | 78,6 | T (*P*=0,0629) |
| *%C2* | 0,98 | 2,0 | 63,2 | 58,8 | 59,4 | T* (*P*=0,020) PxT * (*P*=0,020) |

(suite)

| | R² | SD | Traitement | | | Effets (1) |
|---|---|---|---|---|---|---|
| | | | TSH-MS01 | TSH-MS02 | TSH-MS03 | |
| %C3 | 0,96 | 2,0 | 24,6 | 26,6 | 24,0 | PxT** (P=0,009) |
| %C4 | 0,96 | 0,7 | 8,1 | 9,0 | 9,0 | |
| %C5 | 0,75 | 1,0 | 0,8 | 1,2 | 2,1 | |
| (C2+C4)/C3 | 0,98 | 0,2 | 2,9 | 2,9 | 3,1 | PxT** (P=0,008) |
| pH | 0,74 | 0,2 | 6,7 | 6,7 | 6,8 | |
| D-Lactate (mmol/L) | 0,91 | 0,6 | 4,8 | 4,3 | 5,1 | |
| L-Lactate (mmol/L) | 0,85 | 1,6 | 6,5 | 5,4 | 8,0 | T (P=0,064) |
| L/D | 0,83 | 0,7 | 1,6 | 1,3 | 1,8 | |

(1) niveau de significativité : *p<0,05 ; **p<0,01 T : effet traitement ; PxT : interaction de la période et du traitement

Bien que l'interaction des facteurs « traitement » et « période » a été significative (p<0,05), il a été possible d'observer que lorsque les produits TSH-MS01 et THS-MS03 ont été distribués aux chevaux, la concentration fécale totale en AGV et la concentration fécale en L-Lactate ont été plus élevées (p<0,05), ce qui traduit une activité plus importante de la microflore fécale.

Ces résultats traduisent une augmentation de l'énergie disponible (AGV et L-lactate) pour l'animal. En outre, l'augmentation du L-lactate ou du rapport L-lactate/D-lactate est avantageuse car cette forme de lactate est plus facilement assimilable (par opposition au D-Lactate qui s'accumule dans le sang et provoque des crampes musculaires à l'animal).

En outre, lorsque les produits TSH-MS01 et THS-MS03 ont été distribués aux chevaux, le pourcentage molaire en acétate a été plus important dans les fèces des chevaux (p<0,05), ce qui traduit une activité fibrolytique fécale plus élevée.

Plus spécifiquement, lorsque les chevaux ont reçu le produit TSH-MS01, le pourcentage en acétate a été significativement plus élevé alors que le pourcentage en propionate a été significativement plus faible. Ces résultats traduisent un effet davantage stimulateur du produit TSH-MS01 sur l'activité fibrolytique de la microflore fécale, et donc une augmentation de l'énergie disponible à l'animal. En pratique, le produit TSH-MS01 sera préférentiellement administré à un herbivore monogastrique qui aura un régime pauvre en amidon et riche en fibre. Le produit TSH-MS01 permet à l'animal de tirer davantage profit des fibres végétales et ce, sur une durée plus longue. Le produit TSH-MS01 est particulièrement adapté à des chevaux de randonnée et de loisir.

En revanche, lorsque les chevaux ont reçu le produit TSH-MS03, il est possible de constater une augmentation encore plus importante d'AGV et de L-lactate. Ces résultats traduisent un effet davantage stimulateur de l'activité fermentaire (augmentation des AGV) et de l'activité amylolytique et lacticolytique de la microflore fécale (augmentation du L-lactate). En pratique, le produit TSH-MS03 sera préférentiellement administré à un herbivore monogastrique qui devra fournir un effort important sur une courte durée. Le produit TSH-MS03 reproduit l'effet d'un régime énergétique, riche en amidon, bien que la ration soit riche en fibres. Par conséquent, le produit TSH-MS03 permet à l'animal de tirer davantage profit de sa ration alimentaire et ce, rapidement après absorption de sa ration. Le produit TSH-MS03 est particulièrement adapté à des chevaux de course ou de sport.

Tableau 7 : Coefficient de variation (%) des mesures en AGV, lactate et pH en fonction du traitement reçu par les chevaux (n=6)

| Coefficient de variation (%) | Traitement | | |
|---|---|---|---|
| | TSH-MS01 | TSH-MS02 | TSH-MS03 |
| Total AGV | 33,3 | 30,0 | 45,7 |
| %C2 | 5,0 | 22,7 | 15,3 |

(suite)

| Coefficient de variation (%) | Traitement | | |
|---|---|---|---|
| | TSH-MS01 | TSH-MS02 | TSH-MS03 |
| %C3 | 8,7 | 37,8 | 30,6 |
| %C4 | 23,3 | 30,1 | 20,1 |
| %C5 | 38,1 | 64,5 | 87,9 |
| (C2+C4)/C3 | 10,8 | 41,2 | 34,0 |
| pH | 3,6 | 2,2 | 5,8 |
| D-Lactate | 28,8 | 26,5 | 29,0 |
| L-Lactate | 40,2 | 40,9 | 24,9 |
| L/D | 73,9 | 40,0 | 64,7 |

Pour les mesures d'AGV, le coefficient de variation a été numériquement plus faible lorsque les chevaux ont consommé le produit TSH-MS01. Le produit TSH-MS01 tend à réduire les variations entre les individus. Ceci montre que le produit TSH-MS01 a un effet sur l'apport d'énergie présentant une forte reproductibilité et stabilité.

**2.2.2. Dénombrements microbiens de la flore fécale autochtone**

**Tableau 8 :** Effet des produits TSH-MS01, 02 et 03 sur le dénombrement microbien des flores anaérobie totale, amylolytique, lacticolytique et cellulolytique mesuré dans les fèces de chevaux (n6).

| Flore (log10 cfu/mL) | $R^2$ | SD | Traitements | | |
|---|---|---|---|---|---|
| | | | TSH-MS01 | TSH-MS02 | TSH-MS03 |
| Flore anaérobie totale | 0,91 | 0,3 | 7,3 | 7,1 | 7,4 |
| Flore lacticolytique | 0,58 | 0,6 | 6,6 | 6,7 | 7,0 |
| Flore amylolytique | 0,76 | 0,7 | 5,0 | 5,3 | 5,5 |
| Flore cellulolytique | 0,88 | 17,5 | 5,6 | 4,7 | 4,8 |

Lorsque les chevaux ont consommé le produit TSH-MS01, les concentrations fécales en bactéries anaérobie totales ont été numériquement augmentées, ce qui traduit une activité de la micro flore intestinale plus élevée. Les flores lacticolytique et amylolytique sont légèrement diminuées au profit de la flore cellulolytique, ce qui traduit une activité fibrolytique plus élevée.

Lorsque les chevaux ont consommé le produit TSH-MS03, les concentrations fécales en bactéries anaérobie totales ont été numériquement davantage augmentées, ce qui traduit une activité de la micro flore intestinale encore plus élevée. Les activités lacticolytique, amylolytique et cellulolytique ont été numériquement augmentées, ce qui traduit une activité fibrolytique et amylolytique plus élevée.

Ces résultats confirment les résultats du tableau 6.

**2.3. Digestibilité des constituants de la ration**

Effet des produits MS-TSH01, 02 et 03 sur la digestibilité in vivo des constituants de la ration, en utilisant l'ADL comme marqueur interne (n=6).

Lorsque les produits TSH-MS01 et TSH-MS03 ont été distribués, la digestibilité des constituants fibreux de la ration a été augmentée.

**Tableau 9** : Coefficient de variation (%) des mesures de digestibilité des constituants de la ration en fonction du traitement (n=6)

| Coefficient de variation (%) | Traitement | | |
| --- | --- | --- | --- |
| | TSH-MS01 | TSH-MS02 | TSH-MS03 |
| dMS (%) | 3,7 | 9,0 | 8,0 |
| dMO (%) | 3,0 | 7,8 | 6,8 |
| dNDF (%) | 10,2 | 17,3 | 15,9 |
| dADF (%) | 19,7 | 26,0 | 23,5 |
| dhémicelluloses (%) | 3,9 | 12,7 | 11,8 |
| dcellulose (%) | 16,5 | 22,6 | 20,4 |
| dparois (%) | 8,5 | 10,3 | 15,7 |

Pour l'ensemble des mesures de digestibilité, le coefficient de variation a été numériquement plus faible lorsque les chevaux ont consommé les produits TSH-MS01 et TSH-MS03. Nous pouvons supposer que les produits TSH-MS03 et encore davantage le produit TSH-MS01 tendent à réduire les variations interindividuelles qu'il existe au niveau des mesures de digestibilité. Ceci traduit la reproductibilité et la stabilité des effets de la supplémentation selon l'invention.

**Exemple 2**

Dans cet exemple, nous avons mesuré l'impact d'une supplémentation en TSH-MS01 sur la digestibilité des constituants de la ration avant, pendant et après une épreuve d'endurance.

**Rappel du protocole mis en oeuvre**

**Animaux :** Six chevaux pur-sang arabes (deux juments et quatre hongres) âgés de 7 à 13 ans, d'un poids moyen et d'une note d'état corporel (NEC) de 422,5 kg $\pm$ 20,7 et de 3,2 $\pm$ 0,2 respectivement en début d'expérience ont été utilisés.

Les animaux ont été conduits en boxes individuels (13,3 m$^2$), sur litière artificielle (Copeaux Classic, Thierwhol, Retteinmaier, France). Les chevaux ont été sortis en paddock individuel enherbé quotidiennement de 10h à 17h. Ils ont eu à disposition un bloc de sels minéraux à volonté et un libre accès à un abreuvoir automatique. **Entraînement :** Les chevaux ont été entraînés de façon similaire. L'entraînement a été constitué de sorties montées (2h30), en extérieur sur terrain varié, 3 à 4 fois par semaine. Les entraînements se réalisent au trois allures, sur une distance d'environ 20km et une vitesse moyenne de 10 km/h. Les chevaux ont également été sortis au marcheur quotidiennement (1h à 7 km/h). Des séances spécifiques de galop sur piste ont été réalisées 2 semaines avant une épreuve.

**Compétitions :** Pendant l'expérimentation, les chevaux ont participé à deux compétitions officielles de 130 km (CEI**) au cours de l'année 2009.

**Alimentation :** Pendant toute la durée de l'expérimentation, les chevaux ont reçu une ration à base de foin de prairie naturelle et d'aliment concentré (DP Puissance granulé). La ration journalière a été composée de deux repas de foin, distribués à 10h00 et 16h00, et de deux repas d'aliment concentré, distribués à 08h00 et 17h30. En dehors des périodes expérimentales, les quantités d'aliment concentré distribuées ont été estimées en fonction de l'évolution du poids et de l'état corporel des chevaux de manière à maintenir leur poids de forme. Pendant les périodes de mesures, les même aliments ont été distribués, et les quantités fixées selon un ratio fourrage/concentré de 85/15 et un niveau d'ingestion de 2,6 kg de foin par 100kg de poids vif.

Le jour des épreuves d'endurance, les mêmes aliments ont été distribués mais les rythmes de distribution et les quantités ont été différents : le concentré a été distribué en plusieurs petits repas à chaque vet-gate et le foin distribué à volonté la veille et le soir de la course par exemple.

Une supplémentation en THS - MS01 a été effectuée pendant les trois semaines précédant la course et jusqu'à 13 jours après, selon le dispositif expérimental décrit ensuite. Les doses de produit THS - MS01 (sachets de 2g contenant 1. 10$^{10}$ CFU) ont été préalablement mélangées à 50g farine de tourteau de soja et de maïs avant d'être distribuées aux animaux avec le repas de granulés du matin. Pendant l'épreuve d'endurance, une dose de produit a été distribuée avec chaque repas de concentré.

**Dispositif expérimental** : Les six chevaux ont été répartis en deux lots. Le premier lot (Belik, Riminita et Zaaf) a été supplémenté pendant la première période d'entraînement précédant la première course (P1), l'autre lot (Nafar, Kebar er Naya) constituant le témoin puis nous avons inversé en deuxième période (P2), chaque cheval étant ainsi son propre contrôle.

Les mesures de digestibilité totale apparente ont été réalisées avant, pendant et après deux épreuves d'endurance qui correspondront aux deux périodes expérimentales P1 et P2. Chaque période a débuté 7 jours avant la course et s'est achevé 13 jours après ce qui fait une durée totale de 21 jours.

## Résultats

### Résultats en compétition

Au cours de la première période, cinq des six chevaux ont participé à une épreuve d'endurance de 130 km. Au cours de la deuxième période, les six chevaux ont participé à une épreuve de 130 km.

### Alimentation

Les chevaux ont consommé sans problème les doses de produit tout au long des périodes expérimentales, y compris le jour de la course.

### Digestibilité totale apparente

La digestibilité totale apparente est une mesure qui permet d'évaluer de manière globale l'efficacité des différents processus digestifs sur l'ensemble du tractus digestif. Elle représente le pourcentage d'un aliment d'un de ses constituants ayant disparu au cours de son passage dans le tube digestif, par comparaison de l'ingéré et de l'excrété. Dans notre essai, les mesures de digestibilité ont été réalisées grâce à une collecte partielle des fèces pendant 4 jours consécutifs (Goachet et al., 2009). Ainsi,

- T0:le pool 1 est composé des fèces prélevées de J-7 à J-4 (avant course),
- T7 : le pool 7 est composé des fèces prélevées de J-1 à J+2,
- T8 : le pool 8 est composé des fèces prélevées de J0 à J+3,
- T9 : le pool 9 est composé des fèces prélevées de J+1 à J+4,
- T18 : le pool 18 est composé des fèces prélevées de J+10 à J+13.

Les valeurs de digestibilité obtenues dans cet essai sont conformes à la bibliographie pour un même type de régime (Pagan et al., 1998 ; Goachet et al., 2009).

De manière générale, la digestibilité de la MO et des constituants pariétaux a été plus élevée lorsque les chevaux ont reçu le traitement TSH (tableau 10).

*Tableau 10 : Digestibilité moyenne \* et écart-type* (%) *de la MO et des constituants pariétaux, avec ou sans supplémentation.*

| (n=6) | Control | TSH | P value |
|---|---|---|---|
| dMO | 52,9 ± 3,7 | 56,3 ± 2,7 | 0,001 |
| dNDF | 42,4 ± 5,0 | 46,3 ± 3,9 | 0,003 |
| dADF | 37,4 ± 5,1 | 41,0 ± 3,3 | 0,001 |
| d(NDF-ADF) | 48,0 ± 5,3 | 52,3 ± 4,9 | 0,008 |
| **d(ADF-ADL)** | 42,8 ± 5,8 | 47,0 ± 3,8 | 0,001 |

*\* Moyennes des pools 1, 7 et 18.*

D'autre part, les coefficients de digestibilité de la MO, du NDF et de l'ADF du pool 7 ont été significativement plus élevés avec le traitement TSH (tableau 11).

Le pool 7 correspond à la période autour de la course : transport aller/course/transport retour et 1 journée de repos.

Le tableau 11 montre que les animaux auxquels est administré au moins une souche de bactérie selon l'invention possèdent une digestibilité et une assimilabilité des fibres et des céréales améliorée par rapport au témoin, et cela même en conditions d'effort physique (endurance) et de stress (Pool 7).

*Tableau 11 : Digestibilité moyenne* (%) *de la MO et des constituants pariétaux aux différents pools, avec ou sans supplémentation*

|  |  | *Pool 1* | *Pool 7* | *Pool 8* | *Pool 9* | **Pool 18** |
|---|---|---|---|---|---|---|
| dMO | Témoin | 53,5 | **51,7 a** | 51,6 | 51,8 | 53,4 |
|  | TSH | 55,7 | **56,6 b** *(p≤0,02)* | 55,5 | 55,2 | 56,5 |
| dNDF | Témoin | 42,7 | **40,9** | 40,8 | 41,5 | 43,5 |
|  | TSH | 45,6 | **45,8** *(p≤0,06)* | 44,5 | 44,7 | 47,5 |
| dADF | Témoin | 38,0 | **36,3 a** | 36,5 | 37,6 | 37,9 |
|  | TSH | 41,2 | **39,8 b** *(p≤0,03)* | 39,4 | 39,5 | 42,1 |
| d(NDF-ADF) | Témoin | 48,1 | **46,0** | 45,7 | 46,0 | 49,8 |
|  | TSH | 50,7 | **52,4** | 50,2 | 50,6 | 53,6 |
| d(ADF-ADL) | Témoin | 43,5 | **41,6** | 41,8 | 43,0 | 43,3 |
|  | TSH | 47,0 | **45,7** | 45,3 | 45,3 | 48,2 |

**Revendications**

1.   Procédé pour améliorer la digestibilité et l'assimilabilité des fibres et/ou des céréales chez un animal herbivore monogastrique comprenant l'étape d'administrer audit animal herbivore monogastrique une quantité efficace d'au moins une souche de bactérie choisie dans le groupe constitué des souches de bactéries des genres *Lactobacillus, Lactococcus, Propionibacterium, Bifidobacterium et Bacillus.*

2.   Procédé selon la revendication 1, **caractérisé en ce que** l'étape d'administrer audit animal herbivore monogastrique une quantité efficace d'au moins une souche de bactérie choisie dans le groupe constitué des souches de bactéries des genres *Lactobacillus, Lactococcus, Propionibacterium, Bifidobacterium et Bacillus* consiste à administrer une quantité efficace d'un mélange d'au moins deux souches de bactéries choisies dans le groupe constitué des souches de bactéries des genres *Lactobacillus, Lactococcus, Propionibacterium, Bifidobacterium et Bacillus.*

3.   Procédé selon la revendication 2, **caractérisé en ce que** ledit mélange de souches de bactéries comprend au moins une souche de bactérie du genre *Lactobacillus.*

4.   Procédé selon l'une quelconque des revendications précédentes, **caractérisé en ce que** lesdites souches de bactéries sont choisies parmi les souches de bactéries des espèces *L. paracasei, L. casei, L. acidophilus, L. buchnerii , L. farciminis, L. rhamnosus, L. reuteri, L. fermentum, L. brevis, L. lactis* et *L. plantarum , Lactococcus cremoris* et *Lactococcus lactis ; Propionibacterium jensenii, Propionibacterium acidipropionici, Propionibacterium freudenreichii* et *Propionibacterium freudenreichii* ssp *shermanii; Bifidobacterium longum, Bifidobacterium lactis et Bifidobacterium animalis ; Bacillus licheniformis, Bacillus subtilis* et *Bacillus cereus.*

5.   Procédé selon la revendication 2 ou la revendication 3, **caractérisé en ce que** ledit mélange d'au moins deux souches de bactéries est un mélange d'au moins une souche de *L. plantarum* et d'au moins une souche de *Propionibacterium jensenii.*

6.   Procédé selon la revendication 5, **caractérisé en ce que** ledit mélange est un mélange de *L. plantarum* Lp115 et de *Propionibacterium jensenii* P63.

7.   Procédé selon l'une quelconque des revendications précédentes, **caractérisé en ce que** les souches de bactéries sont inactivées.

**8.** Procédé selon l'une quelconque des revendications précédentes, **caractérisé en ce que** ladite quantité efficace d'au moins une souche de bactérie est administrée audit animal herbivore monogastrique en supplémentant un aliment destiné audit animal avec ladite quantité efficace d'au moins une souche de bactérie.

**9.** Procédé selon l'une quelconque des revendications précédentes, **caractérisé en ce que** ledit animal herbivore monogastrique est choisi parmi les équidés et les suidés, préférentiellement ledit animal herbivore monogastrique est un équidé, plus préférentiellement un cheval ou un poney.

**10.** Supplément alimentaire pour animal herbivore monogastrique comprenant au moins une souche de bactérie choisie dans le groupe constitué des souches de bactéries des genres *Lactobacillus, Lactococcus, Propionibacterium, Bifidobacterium et Bacillus.*

**11.** Supplément alimentaire selon la revendication 10, comprenant au moins une souche de bactérie choisie dans le groupe constitué des espèces *L. paracasei , L. casei, L. acidophilus, L. buchnerii, L. farciminis, L. rhamnosus, L. reuteri, L. fermentum, L. brevis, L. lactis* et *L. plantarum* ; *Lactococcus cremoris* et *Lactococcus lactis ; Propionibacterium jensenii, Propionibacterium acidipropionici, Propionibacterium freudenreichii* et *Propionibacterium freudenreichii* ssp *shermanii ; Bifidobacterium longum, Bifidobacterium lactis et Bifidobacterium animalis ; Bacillus licheniformis, Bacillus subtilis et Bacillus cereus.*

**12.** Supplément alimentaire selon la revendication 11, comprenant au moins une souche de bactérie choisie parmi *L. plantarum* Lp115 et *Propionibacterium jensenii* P63.

**13.** Aliment pour animal herbivore monogastrique **caractérisé en ce qu'**il est supplémenté avec un supplément alimentaire selon l'une quelconque des revendications 10-12.

**14.** Utilisation du supplément alimentaire selon la revendication 12 pour réguler la glycémie chez un animal herbivore monogastrique.

**15.** Utilisation d'une souche de bactérie choisie dans le groupe constitué des souches de bactéries des genres *Lactobacillus, Lactococcus, Propionibacterium, Bifidobacterium et Bacillus* pour diminuer chez un animal herbivore monogastrique les perturbations de la digestion induites par un exercice physique intense et/ou un stress.

**FIG. 1**

Europäisches
Patentamt

European
Patent Office

Office européen
des brevets

**RAPPORT DE RECHERCHE EUROPEENNE**

Numéro de la demande

EP 10 15 7431

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | ANONYMOUS: "Equine Nutrilak Plus" AGRI-GROWTH INTERNATIONAL, [Online] 5 novembre 2007 (2007-11-05), pages 1-3, XP002539050 Extrait de l'Internet: URL:http://web.archive.org/web/20071105011 540/http://www.agriorganics.com/horsecare/ horseprobiotic.html> [extrait le 2009-07-27] | 1-4, 8-11,13 | INV. A23K1/00 A23K1/18 |
| A | * le document en entier * | 14,15 | |
| X | ANONYMOUS: "Horse Feeds UK Nutrition & Health For Your Horse" HORSE FEEDS, [Online] 7 décembre 2007 (2007-12-07), pages 1-2, XP002539051 Extrait de l'Internet: URL:http://web.archive.org/web/20071207163 241/http://www.horsefeeds.co.uk/probiotics .html> [extrait le 2009-07-27] | 1-4,9 | |
| A | * le document en entier * | 8,10,11, 13-15 | A23K |
| X,D | US 6 887 489 B2 (REHBERGER THOMAS [US] ET AL REHBERGER THOMAS [US] ET AL) 3 mai 2005 (2005-05-03) | 10,11,13 | |
| A | * revendications 6-9 * * colonne 4, ligne 24,25 * | 1-9 | |
| X | US 2007/298013 A1 (ALTMAN JAY A [US]) 27 décembre 2007 (2007-12-27) * alinéas [0008], [0010], [0011], [0017] * * revendication 1 * | 1-4,8, 10,11,13 | |

DOMAINES TECHNIQUES RECHERCHES (IPC)

-/--

Le présent rapport a été établi pour toutes les revendications

| Lieu de la recherche | Date d'achèvement de la recherche | Examinateur |
|---|---|---|
| Munich | 19 mai 2010 | Barac, Dominika |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

# RAPPORT DE RECHERCHE EUROPEENNE

Numéro de la demande

EP 10 15 7431

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | Anonymous: "Welcome to : Ultra Bio-Logics Inc. Equine Health Through Balanced Nutrition." Ultra Biologics 21 septembre 2007 (2007-09-21), pages 1-20, XP002582790 Extrait de l'Internet: URL:http://web.archive.org/web/20070921112 558/http://www.ublcorp.com/files/Equinecar e.pdf [extrait le 2010-05-18] | 1-4, 8-11,13 | |
| A | * Equine DW-123 Equine Nutrilak Plus * | 7,15 | |
| | ----- | | |
| A | DATABASE WPI Section Ch, Week 200923 Thomson Scientific, London, GB; Class B04, AN 2009-F18349 XP002539052 & KR 2008 107 193 A (UNIV IND COLLABORATION & CONSULTING) 10 décembre 2008 (2008-12-10) * abrégé * | 1,14 | |
| | ----- | | DOMAINES TECHNIQUES RECHERCHES (IPC) |
| A | CN 1 559 292 A (HAIDA BIOLOG TECH CO LTD NANYA [CN]) 5 janvier 2005 (2005-01-05) * abrégé * | 1,14 | |
| | ----- | | |
| A | US 4 981 705 A (TOMES NANCY J [US]) 1 janvier 1991 (1991-01-01) * colonne 4, ligne 28-41 * * revendications 1,5-7 * | 5,6,12 | |
| | ----- | | |
| | -/-- | | |

Le présent rapport a été établi pour toutes les revendications

| Lieu de la recherche | Date d'achèvement de la recherche | Examinateur |
|---|---|---|
| Munich | 19 mai 2010 | Barac, Dominika |

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 10 15 7431

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | DATABASE WPI<br>Section Ch, Week 200868<br>Thomson Scientific, London, GB;<br>Class B04, AN 2008-L61440<br>XP002582791<br>& JP 2008 195635 A (CROSSFIELD BIO KK)<br>28 août 2008 (2008-08-28)<br>* le document en entier *<br>----- | 15 | |

DOMAINES TECHNIQUES
RECHERCHES (IPC)

Le présent rapport a été établi pour toutes les revendications

| Lieu de la recherche | Date d'achèvement de la recherche | Examinateur |
|---|---|---|
| Munich | 19 mai 2010 | Barac, Dominika |

CATEGORIE DES DOCUMENTS CITES

X : particulièrement pertinent à lui seul
Y : particulièrement pertinent en combinaison avec un
　 autre document de la même catégorie
A : arrière-plan technologique
O : divulgation non-écrite
P : document intercalaire

T : théorie ou principe à la base de l'invention
E : document de brevet antérieur, mais publié à la
　 date de dépôt ou après cette date
D : cité dans la demande
L : cité pour d'autres raisons
...............................................................................................
& : membre de la même famille, document correspondant

EPO FORM 1503 03.82 (P04C02)

Europäisches
Patentamt

European
Patent Office

Office européen
des brevets

Numéro de la demande

EP 10 15 7431

## REVENDICATIONS DONNANT LIEU AU PAIEMENT DE TAXES

La présente demande de brevet européen comportait lors de son dépôt les revendications dont le paiement était dû.

☐ Une partie seulement des taxes de revendication ayant été acquittée dans les délais prescrits, le présent rapport de recherche européenne a été établi pour les revendications pour lesquelles aucun paiement n'était dû ainsi que pour celles dont les taxes de revendication ont été acquittées, à savoir les revendication(s):

☐ Aucune taxe de revendication n'ayant été acquittée dans les délais prescrits, le présent rapport de recherche européenne a été établi pour les revendications pour lesquelles aucun paiement n'était dû.

## ABSENCE D'UNITE D'INVENTION

La division de la recherche estime que la présente demande de brevet européen ne satisfait pas à l'exigence relative à l'unité d'invention et concerne plusieurs inventions ou pluralités d'inventions, à savoir:

voir feuille supplémentaire B

☐ Toutes les nouvelles taxes de recherche ayant été acquittées dans les délais impartis, le présent rapport de recherche européenne a été établi pour toutes les revendications.

☒ Comme toutes les recherches portant sur les revendications qui s'y prêtaient ont pu être effectuées sans effort particulier justifiant une taxe additionnelle, la division de la recherche n'a sollicité le paiement d'aucune taxe de cette nature.

☐ Une partie seulement des nouvelles taxes de recherche ayant été acquittée dans les délais impartis, le présent rapport de recherche européenne a été établi pour les parties qui se rapportent aux inventions pour lesquelles les taxes de recherche ont été acquittées, à savoir les revendications:

☐ Aucune nouvelle taxe de recherche n'ayant été acquittée dans les délais impartis, le présent rapport de recherche européenne a été établi pour les parties de la demande de brevet européen qui se rapportent à l'invention mentionnée en premier lieu dans les revendications, à savoir les revendications:

☐ Le present rapport supplémentaire de recherche européenne a été établi pour les parties de la demande de brevet européen qui se rapportent a l'invention mentionée en premier lieu dans le revendications (Règle 164 (1) CBE)

Europäisches
Patentamt

European
Patent Office

Office européen
des brevets

**ABSENCE D'UNITÉ D'INVENTION
FEUILLE SUPPLÉMENTAIRE B**

Numéro de la demande

EP 10 15 7431

La division de la recherche estime que la présente demande de brevet européen ne satisfait pas à l'exigence relative à l'unité d'invention et concerne plusieurs inventions ou pluralités d'inventions, à savoir :

1. revendications: 1-9

   Procédé pour améliorer la digestibilité et assimilabilité des fibres chez un animal monoqastrique
   ---

2. revendications: 10-14

   Supplément/aliment pour des animaux monogastriques comprenant une souche de bactérie et l'utilisation de ce supplément/aliment
   ---

3. revendication: 15

   Utilisation d'une souche de bactérie pour diminuer les perturbation de la digestion chez un animal monogastrique après d' exercice physique et/ou du stress
   ---

EPO FORM P0402

**ANNEXE AU RAPPORT DE RECHERCHE EUROPEENNE
RELATIF A LA DEMANDE DE BREVET EUROPEEN NO.**

EP 10 15 7431

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

19-05-2010

| Document brevet cité au rapport de recherche | | Date de publication | Membre(s) de la famille de brevet(s) | | Date de publication |
|---|---|---|---|---|---|
| US 6887489 | B2 | 03-05-2005 | US | 2003007953 A1 | 09-01-2003 |
| | | | US | 6455063 B1 | 24-09-2002 |
| US 2007298013 | A1 | 27-12-2007 | WO | 2007150052 A1 | 27-12-2007 |
| KR 2008107193 | A | | AUCUN | | |
| CN 1559292 | A | 05-01-2005 | AUCUN | | |
| US 4981705 | A | 01-01-1991 | AT | 400282 B | 27-11-1995 |
| | | | BE | 1002673 A3 | 30-04-1991 |
| | | | CZ | 9005193 A3 | 15-11-1995 |
| | | | DD | 293483 A5 | 05-09-1991 |
| | | | DE | 4009999 A1 | 08-05-1991 |
| | | | DK | 68090 A | 07-05-1991 |
| | | | FR | 2653973 A1 | 10-05-1991 |
| | | | GB | 2237723 A | 15-05-1991 |
| | | | JP | 2516452 B2 | 24-07-1996 |
| | | | JP | 3147751 A | 24-06-1991 |
| | | | NL | 9000676 A | 03-06-1991 |
| | | | NO | 901296 A | 07-05-1991 |
| | | | SK | 519390 A3 | 11-01-1999 |
| JP 2008195635 | A | 28-08-2008 | AUCUN | | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6951643 B
- US 7470531 B
- US 5534271 A
- US 552973 A
- US 6887489 B
- US 6455063 B

**Littérature non-brevet citée dans la description**

- **J. Scott Weese ; Joyce Rousseau.** *JAVMA,* 15 Juin 2005, vol. 226 (12
- **Yuyama et al.** Evaluation of a host-specific Lactobacillus probiotic in neonatal foals. *J Appl Res Vet Med,* 2004, vol. 2, 26-33
- **Glade, M. J.** *Journal of Equine Veterinary Science,* 1991, vol. 11 (1), 10-16
- **Glade, M. J.** *Journal of Equine Veterinary Science,* 1991, vol. 11 (6), 323-329
- **Glade, M. J.** *Supplement to proceedings of Alltech's eighth annual symposium,* 1992, 1-26
- **Glade, M. J. ; L. M. Biesik.** *Journal of Animal Science,* 1986, vol. 62, 1635-1640
- **Glade, M. J. ; M. D. Sist.** *Nutrition Reports International,* 1998, vol. 37, 11-17
- **Hall, R. R. ; S. G. Jackson et al.** *Journal of Equine Veterinary Science,* 1990, vol. 10 (2), 130-134
- **Hausenblasz, J. ; J. Szuco et al.** *9th Biotechnology in the Feed Industry symposium, Lexington, KY, Alltech Technical Publications,* 1993
- **Hill, J. ; S. Gutsell.** *BSAS annual meeting,* 1998
- **Kim, S. M. ; C. M. Kim et al.** *Korean Journal of Animal Nutrition and feedstuff,* 1991, vol. 15 (5), 272-280
- **Medina, B.** *Dijon, France, Université de Bourgogne,* 2003, 159
- **Moore, B. E. ; K. E. Newman.** *Journal of Animal Science,* 1994, vol. 72 (1), 261
- **Pagan, J. D.** *Journal of Animal Science,* 1990, vol. 68 (1), 371
- **Goachet et al.** *Comparative Exercise Physiology,* vol. 5 (3-4), 143-151
- **Jouany JP ; Senaud J.** *Reprod Nutr Dev.,* 1982, vol. 22 (5), 735-52